# EUROPEAN PATENT APPLICATION

(11) **EP 1 683 506 A1**
(43) Date of publication of application: **26.07.2006**
(21) Application number: 04791367.8
(22) Date of filing: 18.10.2004
(51) Int. Cl.: A61J 1/12, B65D 85/50

(54) **PROTECTIVE DEVICE FOR THE TRANSPORT OF CLINICAL SAMPLES AND SIMILAR**

(30) Priority: 16.10.2003 ES 200302350 U; 17.11.2003 ES 200302641 U; 08.07.2004 ES 200401723 U; 02.09.2004 ES 200402122 P
(71) Applicant: Sanchez Taboas, Jorge Horacio, E-36203 Vigo (ES); Sanchez Taboas, Ignacio Javier, E-36203 Vigo (ES); Sanchez Taboas, Marcos José, E-36203 Vigo (ES)
(72) Inventor: Sanchez Taboas, Jorge Horacio, E-36203 Vigo (ES); Sanchez Taboas, Ignacio Javier, E-36203 Vigo (ES); Sanchez Taboas, Marcos José, E-36203 Vigo (ES)
(74) Representative: Ungria Lopez, Javier
(86) International application number: PCT/ES2004/000447
(87) International publication number: WO 2005/037186

(57) **Abstract**

It is defined by a rack supporting test tubes, a secondary container with cover wherein the rack is inserted and a tertiary or third cover wherein the secondary container is likewise inserted.

The rack has small hollow recesses or perforations wherein the test tubes or primary containers containing the clinical samples fit.

The equipment is leakproof and capable of dampening impacts with support devices for refrigerants and documents.

It is made out of materials capable of enduring autoclaving and/or chemical disinfection.

Besides, it is capable of also enduring differences of pressure when it is used in transport by air or with carbon dioxide snow type refrigerants.

## Description

### OBJECT OF THE INVENTION

As expressed in the title of this specification, the present invention refers to protective equipment for transporting clinical samples and the like, whose purpose is to be formed like a surface perimetric covering, and on the bottom part, built to cover and protect the bodies which are to facilitate the transporting of organic samples included in a part prepared to keep the sample holders in a certain safe position. The invention is formed as a protective part of a primary container of the sample itself, a secondary container, holding the sample in a suitable and safe position, upon which the protective container or cover that acts as an outside container that will act as a support of the primary and secondary container, as well as of the outside container, is included.

Besides, the equipment of the invention will be able to keep the temperature controlled.

On the other hand, the rack that holds the test tubes comprising the primary or other containers for transporting clinical samples, is designed in order to achieve, from said rack, absorption of a possible leak of the contents of the containers of samples or materials, leaks that may be caused by the knocking or breakage of the containers contained inside the containers.

### FIELD OF THE INVENTION

This invention is useful in the industry dedicated to manufacturing apparatus, devices, parts and accessories for protecting containers, and especially containers used for transporting products useable as medical samples and the like in test tubes for clinical or pharmaceutical use.

### BAKCGROUND OF THE INVENTION

Conventionally, primary containers or racks are structured from a body that forms a type of tray or container, with its side walls open for the most part for the purpose of facilitating visualization thereof as far as the tubes included inside them are concerned. They also make the test tubes accessible and the bottom thereof is grated. All the racks are provided with a grating size sufficient to define a support for the bottom end of the cited test tubes. A pair of grated parts shaped like quadrangular section grids is established in the center of the container. These grids may be round or have any other shape that is considered convenient and they may be fastened as a monobloc or single piece to the side walls of the container or else they may be capable of being disassembled from the general assembly, just as in the case of modular racks that are being used nowadays.

In all cases, the racks are capable of complying with their function as a support element of the test tubes vertically as well as horizontally, but currently there is no knowledge of the existence of an invention which, shaped like a rack, is capable of absorbing the fluids contained in the test tubes in the event that they accidentally break.

In all cases, some are made like shelving provided with shelves, others are like baskets with a cover and they have different capacities of being disassembled.

It has been confirmed that nowadays different racks made out of different materials, such as polypropylene, rigid in all cases and of non-absorbent quality, polyester foam with absorption capacity, polystyrene without absorption capacity, rigid acrylic materials, cardboard, acetal, polycarbonates and even stainless steel, have been developed.

All the racks made at present have the purpose of holding the tube containing the sample inside its structure. The applicant has no knowledge of the present existence of a rack used specifically apart from holding test tubes, for holding the liquids spilt by a leak, breakage or spilling of the liquid materials transported inside the tubes included in the inside of the structure of the rack.

In connection with test tubes held by the rack, there is no knowledge of tubes with the characteristics that are proposed, although it is true that there are different models of tubes with a similar outside appearance on the market, but they are not capable of enduring a differential pressure equal to or higher than 0.95 bars. The market study has shown the existence of test tubes with special plugs and different shapes, but none of them have the capacity to endure said inside differential pressure and with a safety structure to resist very strong impacts.

### DESCRIPTION OF THE INVENTION

The protective equipment for transporting clinical samples and the like that constitute the object of the invention is comprised of a body that will have the perimetric shape in accordance with the secondary container, which will not have a circular section and in turn it is with the rack holding the samples. In the bottom part of the pertinent base of the secondary container and in the top part of a cover that permits the inclusion of the holding assembly of the samples and/or rack there is a so-called secondary container, which will inevitably be inserted in the outside container, in order to later allow it to be closed up with the corresponding cover and subsequently inserted as a container that will protect the formed assembly as the inside holding part of the rack, or secondary container, with its samples and the perimetric part with its base and the protective cover of the assembly.

The rack has the particularity of being absorbent and it is formed liked a part capable of carrying out two harmonious functions, in other words, on the one hand, holding the test tubes containing the sample and at the same time, holding and collecting the fluids contained in the test tube in the event that the structure of the tube itself may break.

More specifically, the rack that has an absorbent structure is formed of absorbent materials, just as a soft absorbent polyethylene fiber may be, by means of which a block may be formed or by the superposition of the fibers supplied in sheets, a structure forming an outside container will provide greater resistance to the structure.

After superposing the sheets or parts comprising the block of fiber, the different holes will be made in the general structure with appropriate sizes in order to hold the test tubes or smaller containers to be transported. The general block may be formed by means of a single body with perimetric protective parts or may include protective parts in the top, side or bottom part. It is even possible to form same by means of an assembly of parts that constitute the block.

The rack is capable of having a cover, which is or is not made of absorbent material, provided with recesses or perforations in order to achieve perfect coupling of the tubes or containers to be transported, and the sizes will be determined in accordance with the diameters of the tubes existing on the market. The general body of the rack may have the corresponding featheredgings that smooth out the edges thereof and, at the same time, the rack may be formed by following the same general characteristics, having any physical shape that is considered appropriate, including a cylindrical shape.

Consequently, the rack is formed like an absorbent body improved with respect to the ones existing up to now, that have the basic purpose of achieving absorption of the fluids contained in the test tubes and container with diagnostic samples, included inside the container, used for transporting them.

Other characteristics of the invention refer to the tertiary container or vessel and also to the second container.

Hence, the outside container includes one or several handles for transporting it. The handle will be able to be arranged as an independent part included in the outside container, or else a part of the structure itself of the container. The handle will be able to be arranged on the body of the container or on the cover. Introducing one or several ox-eyes, or the like in order to be able to check the inside of the assembly in case there is a leak in the containers will comprise another improvement. Another improvement is the introduction of an autoclavable and/chemically disinfectible material in case there is a leak inside the secondary container. Another improvement of this container is the introduction of a document holder hollow space on the outside thereof. Another improvement is the inclusion of some side inside supports for the placement of cooling devices. Another improvement is the inclusion of a support for the inclusion of a temperature measuring and recording system. Another improvement is the inclusion inside of some supports for the insertion of the documents of the shipment of test requests and others. Another improvement is the inclusion of a sealing support so that it may not be tampered with or to check that it has not been opened. Another characteristic is that the container includes some means for gripping to other containers and/or devices in the vehicle by means of anchoring in the body or recesses. Another characteristic is to provide the container with some transporting wheels in the base thereof. Another characteristic will be to insert a valve for controlling the difference of pressure when being transported by air or when carbon dioxide snow type refrigerants are used. Another characteristic is the inclusion of an open or closed indicator. Another characteristic is the inclusion of an indicator whether or not there is material inside. Another characteristic is the inclusion of position and risk indicators. Another characteristic is the inclusion of side reinforcements to protect against knocks. This container will have a polygonal section with any number of sides with a minimum of three sides, depending on the shape of the secondary container. Another improvement is the manufacturing of this container in such a way that it is leakproof when carbon dioxide snow is used.

With reference to the secondary container, inevitably included in the anterior container, which will not have a circular section and will have a polygonal section with any number of sides with a minimum of three. In the bottom part of its pertinent base, and in the top part there will be a cover, which will be able to be included in the assembly in a collapsible manner or independently from the assembly that permits the inclusion of the holding assembly of the samples and/or racks, in order to allow same to be closed. This will protect the rack and the samples. Another improvement consists of it being possible for there to be one or several secondary non-circular section containers, which will be able to be inserted inside the cited outside or tertiary container. Another improvement is the direct inclusion of absorbent material in the bottom, side walls and base thereof without the need of using a special rack. This material will be able to be directly inserted by the user himself or may form part of the manufacturing process itself of this secondary container. Another improvement will be the capacity to endure the inside pressure of the container. Another improvement is the insertion of materials with a capacity to be autclavable and/or chemically disinfectible due to a break. Another improvement is the inclusion of some recesses for coupling to other containers or to the outside container. Another improvement is the inclusion of some handles or couplings for the moving thereof.

On the other hand, other important characteristics of the invention refer to the test tubes comprising the primary containers, each one of which is defined as of a tube-shaped body which may or may not be transparent, made out of plastic materials or the like and closed by one of its ends by means of a semi-spherical base that has its convexity towards the outside or another polyhedral shape. A screw plug, anchored to the mouth of the tube, either at the inside since the tube has inside thread or else at the outside in the mouth thereof stops the other end up. The plug may be of a reversed cup type with inside thread. In both cases solid fastening of the plug is obtained in order to endure the cited pressure. The material, which the tube is made out of, endures, once stopped up, the cited differential pressure, in a defined temperature range. It will be thick enough to endure this pressure depending on the manufacturing process and the materials used and it will also be capable of enduring very strong impacts. The outside shape may have, aside from the thread in order to couple the plug, an end of stroke of the thread. Depending on the use and shape of the plug, it may have a pressure joint for the coupling thereof and to improve the gripping thereof to guarantee pressurization. This plug may include the thread in order to be coupled inside the tube, wherein a pressurization and/or leakproof joint has been included. The plug has also been designed to include a perforable primary plug and afterwards to secure the assembly with a secondary plug threaded to the outside of the test tube in order to guarantee pressurization. It has also been provided for that the plug can axially include a brush and also a protective cover that covers the outside part of the plug and adapts to the contour of the tube-shaped body.

The tube-shaped body of the test tube may be cylindrical or slightly conical, open towards the mouth. It may also have a prismatic or polyhedral shape in general.

Hereinafter to provide a better understanding of this specification and forming an integral part thereof, some figures wherein the object of the invention has been represented in an illustrative and non-restrictive manner are attached hereto.

### BRIEF DESCRIPTION OF THE FIGURES

Figures 1 to 3 show different perspective exploded views of the protective equipment for transporting clinical samples and the like, object of the invention. It basically comprises a rack that holds some test tubes primary containers of the clinical samples, a secondary container where the rack with the test tubes is housed and a tertiary or outside container where the assembly of the secondary container is located.
Figure 4 shows a perspective view of the secondary container.
Figure 5 shows a sectional view of the primary container.
Figure 6 shows a sectional view of the secondary container, inside of which the rack holding several test tubes is housed.

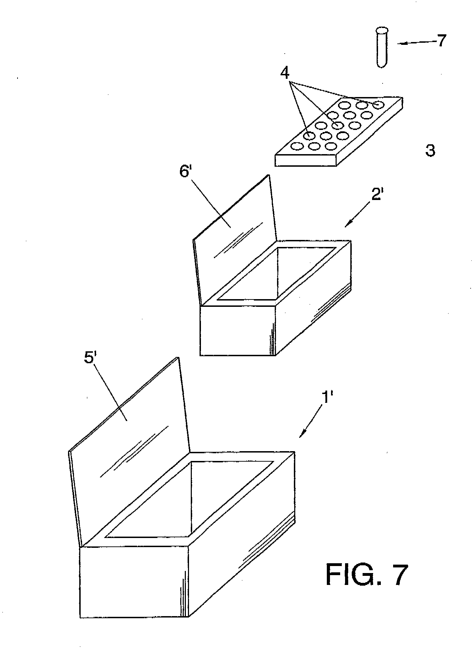

Figure 7 shows another exploded perspective view of the equipment of the invention.

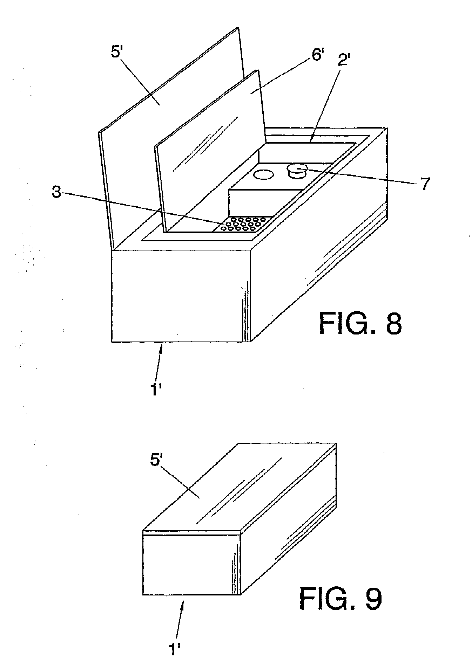

Figure 8 shows a perspective view of the equipment represented in the preceding figure.
Figure 9 shows a view of the equipment represented in the two preceding figures in a totally closed position.
Figure 10 shows a perspective view of the main body of the rack with different housings for placement of the test tubes.
Figure 11 shows a perspective view of an auxiliary part comprising a cover applicable complementally on the side structure of the main body of the rack represented in the preceding figure.
Figure 12 shows the main body of the rack represented in figure 10 wherein a perimetric or semi-perimetric protection of the rack body has been added.
Figures 13 to 21 represent different embodiments of test tubes.

### DESCRIPTION OF THE PREFERRED EMBODIMENT

Considering the numbering used in the figures, the protective equipment of the invention is defined as of a body that can be manufactured out of different materials, such as products derived from oil, such as parts made out of rigid materials or the like, or others, such as, for example, polyethylene, polypropylene, ABS, polycarbonate or other rigid metallic materials, which may or may not be limited to aluminum. The equipment can also be made out of non-rigid materials, which may or may not be limited to polyethylenes, canvas or other non-rigid materials. A11 these materials other than cardboard form a perimetric belt of the body of the secondary container (2, 2') that contains the supports or racks (3), having perforations (4, 4') wherein the parts or test tubes (7) including the samples are inserted.

In turn, the secondary container will be located inside a tertiary or outside container (1, 1'), both having respective closing covers (5, 5' and 6, 6') in order to close the area of access of the equipment assembly for the purpose of preserving the clinical samples, which are inside the test tubes or containers of solid or fluid samples (7).

Hence, it is an assembly of perimetric coverings for the outside area of the sides, as well as for the top and bottom area, of a container wherein a secondary container with a cover will be inevitably included. The test tubes or supports, or racks (3) will be deposited in this secondary container. By means of their vertical perforations (4, 4'), the racks hold fluid samples comprising the samples of materials of different uses. The samples remain included in the context of the primary container (7) formed as a rack made of absorbent material, of a part capable of protecting the perimeter of the rack assembly.

In short, the assembly of the equipment of the invention is formed as a container comprised of three parts comprised of a support or rack (3) for the containers of samples (7), a rigid and leakproof secondary container (2, 2') with a non-circular section and any type of seal and an outside container (1, 1') provided with the above-cited respective cover.

The primary container or test tube (7) assembly will be placed in the secondary container in the racks with absorbent capacity, in such a way that there is no contact at all between the different primary containers (7), thus avoiding them from being able to break or crack and spill their contents on the secondary container.

This secondary container will be placed in the outside container, in such a way that there is filling material to dampen possible impacts or knocks.

The assembly is formed as a container capable of keeping the contents of the primary container comprised of the tubes containing the samples (7) included in the absorbent rack (3) perfectly protected and at a controlled temperature, if necessary. Other characteristics of the invention refer to some improvements of the secondary and tertiary containers.

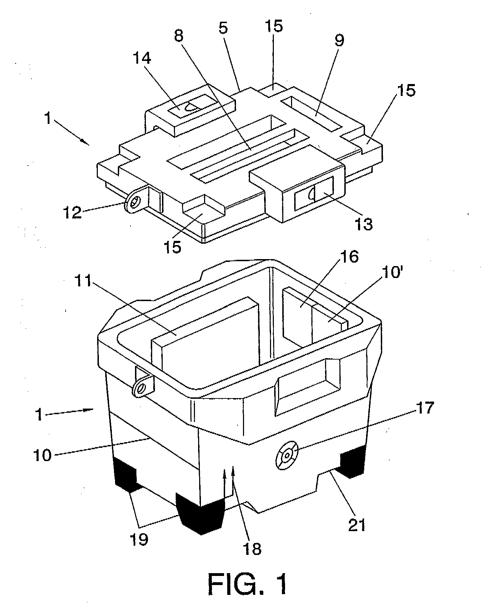

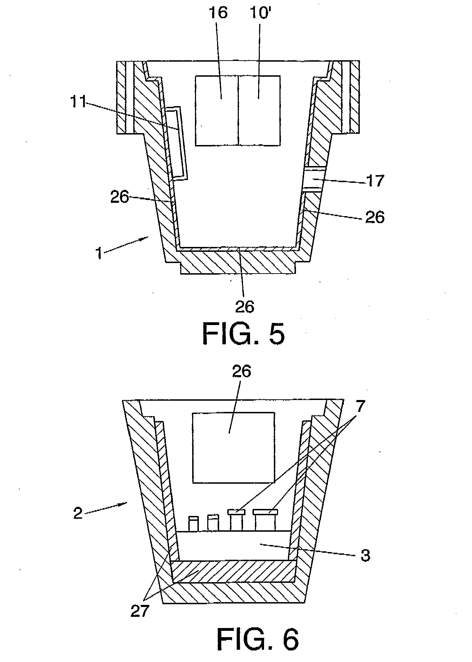

Referring to figures 1 and 5, the cover (5) of the tertiary or outside container (1) includes a handle (8) that forms an integral part of the cover itself (5), an ox-eye (9) like a window, a hook (12) for seals, a device (13) for information whether it is open or closed, a device (14) for information about the existence of material and some recesses (15) for the stacking thereof. These recesses are complemented by the recesses (21) located in the bottom of the body of the tertiary container in question.

The body of this tertiary container (1) includes an outside document holder (10), an inside document holder (10'), an inside refrigerant holder (11), a holder (16) for the temperature controller, a pressure valve (17), some position and risk indicators (18), some corner reinforcements (19), an inside impact dampener (20), as well as the grooves (21) for the coupling stacked on the base that are complementary with the above-cited recesses (15) of the cover (5).

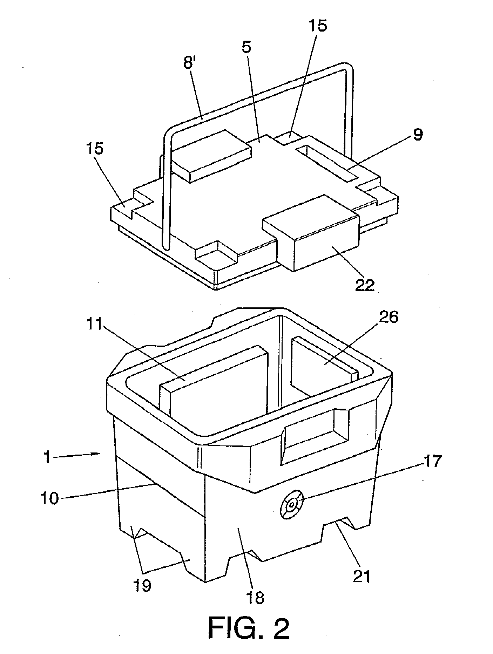

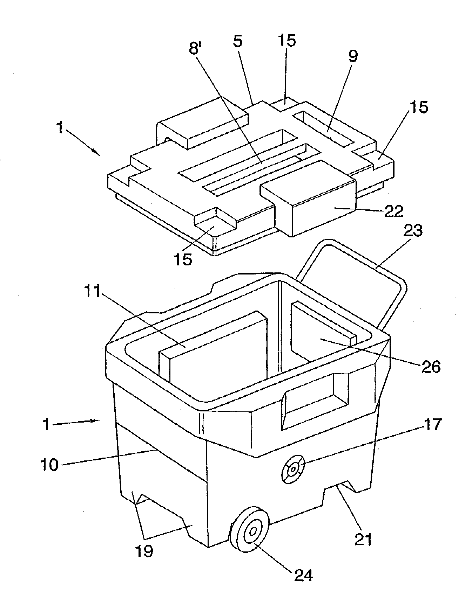

As shown in figures 2 and 3, the cover (5) of the tertiary container (1) includes a collapsible outside handle (8') and a seal (22). In turn the body of the tertiary container (1) includes a collapsible handle (23) so that it may be moved with the help of some wheels (24) that the container in question has in its base.

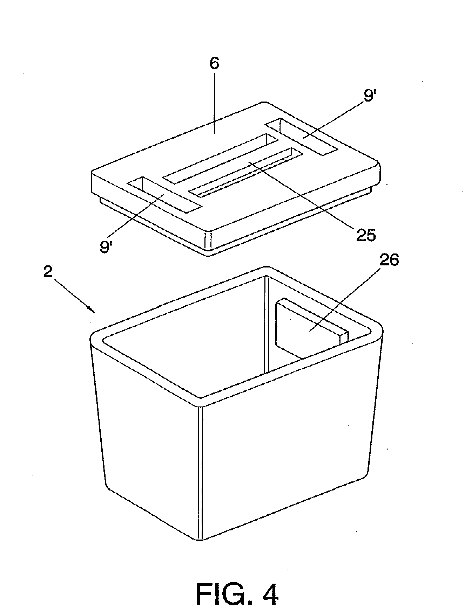

Now referring to figures 4 and 6, one can see the representation of the secondary container (2) with its cover (6) that includes a center handle (25) forming an integral part of said cover (6), some ox-eyes (9') like windows so that one can check the condition of the samples.

The body of this secondary container (2) includes a holder (26) for documents and/or the temperature measuring device. The inside of this secondary container also includes absorbent material (27) that covers the inside surface of the side walls and bottom, without the need of a special rack.

Hence, considering the embodiment shown in figures 1 to 6, we have safer equipment due to the inclusion of pressure valves (17) in order to control the pressure during transport by air and for the use of carbon dioxide snow type refrigerants. We also have a secondary container capable of enduring inside pressures with greater side reinforcements due to the continuous use thereof; safety indicators so that the user knows whether there are samples inside, due to the fact that it is not possible to see the existence of tubes in secondary containers without the opening thereof; the inclusion of ox-eyes or the like in order to check for the existence of leaks in the materials, due to the contaminating potentiality of these samples and due to the inclusion of a cooling system defined in the secondary container; the addition of a sealing system thereof for the safety of the people and the documents included therein according to the law regarding the protection of data considered to be confidential information. Besides, this is all complemented by recesses and notches so that two or more pieces of equipment may be stacked. We also have the inclusion of top and inside holders in order to be able to gather the documents along with the primary containers, such as for example, test requests, or others, and the documents of the shipment, delivery notes or others. Besides the container has handles so that it is easy to move it.

Hence, the equipment of the invention has some improvements of the secondary and tertiary containers of the assembly of said equipment formed by the three basic parts comprised of the rack for holding the test tubes, the leakproof secondary container with the capacity to endure inside pressure with a polygonal section and a seal of the traction, impact, pressure type or the like, but not limited thereto. There is an outside container that acts like a support of the primary container and of the secondary container, as well as an outside protector.

The primary container will be inserted in the secondary container in racks, in such a way that there is no contact at all between the different primary containers. Consequently, this prevents the primary containers from breaking, cracking and spilling their contents in the secondary container.

This secondary container will be placed in the outside container, in such a way that there is insertion of filling material (20) in order to dampen possible impacts or knocks.

The assembly is formed as a container capable of keeping the assembly of the primary container perfectly cooled.

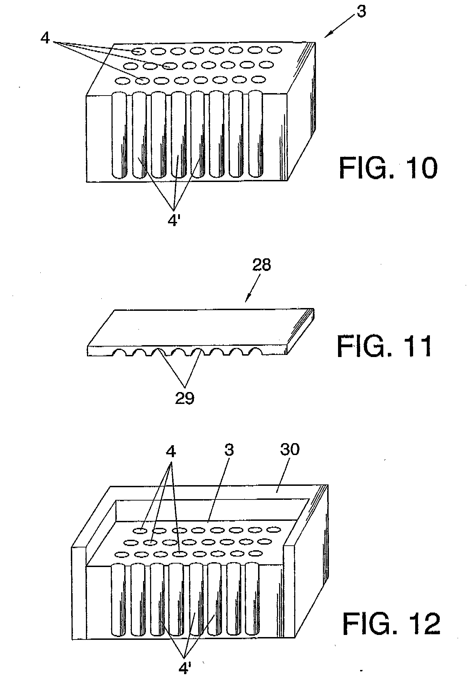

On the other hand, referring to figures 10 to 12, one can see therein an absorbent rack (3) made of an absorbent material, comprising the general body that can have any physical shape that is considered appropriate, such as for example a prism, cylinder, oval-plan body or the like. The fact that the main body is made out of polypropylene microfibers, fiberglass, polypropylene polyester, treated cotton, absorbent cellulose, absorbent paper or other non-rigid absorbent materials exists as basic characteristics of the body itself. The body has vertical perforations (4) and even vertical recesses (4') with a concave shape that, in collaboration with an auxiliary part (28) as a flat cover on one of its larger sides and provided with other recesses (29) on its opposite larger surface, will define a cover of the concave recesses (4') allowing the insertion of test tubes used for transporting clinical samples and fluids.

The rack may be made as a monobloc body or as different bodies coupled together with a similar plan view, irrespective of these bodies producing the existence of a prismatic body or an oval body or else a cylindrical body, as long as its structure has perforations that are harmonious with each other for the insertion of the test tubes. It should be pointed out that the duly formed invention does not have sharp edges and its entire perimeter has smooth edges that prevent the existence of areas that can cause damage when transporting the general contents of the test tubes. The invention is formed like a general body that can have any shape and that can be formed by a monobloc body, provided with perforations (4) and even the recesses (4') overlapped by the cover-type (28) auxiliary part. This cover formed by the flat body and the recesses (29) existing on the surface opposite the outside surface can be perimetrically covered by a rigid material surrounding body (30), that will provide greater impact resistance in the event that there is an accidental knock of the rack body or bodies (3) by an element that can damage the contents of the test tubes contained inside the cavities formed by the perforations (4).

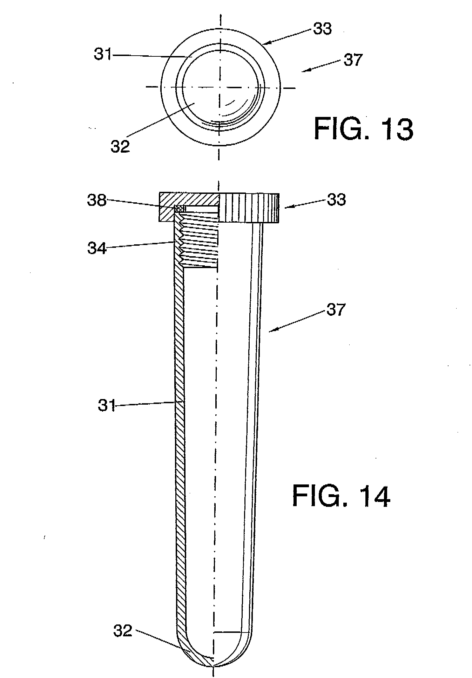

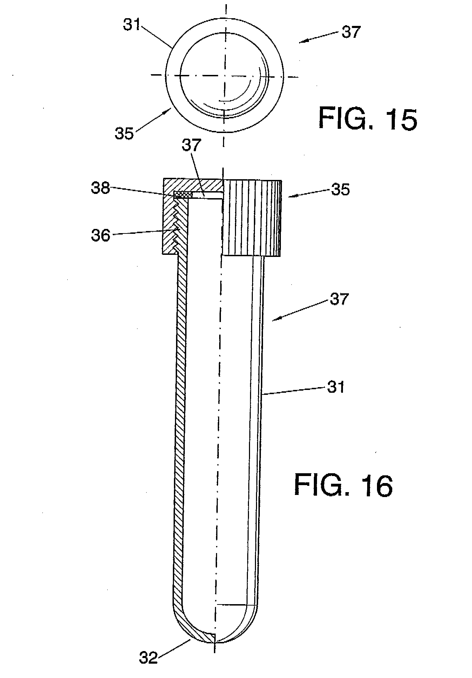

Figures 13 to 21 describe the test tube (7) that is defined by a tube-shaped body (31) with a cylindrical shape (figure 15) or slightly conical shape that opens toward the outside or mouth (figure 13). The blind end (32) ends in a preferably semi-spherical shape, although it could have any other polyhedral shape.

The mouth of the tube-shaped body (31) receives by screwing a solid plug in order to thus be able to endure the required inside pressure. In the case shown in figure 14, the plug has reference number (33) and has a thread on its outside wall. Reference (34) designates the inside thread of the mouth of the tube-shaped body (31). In this embodiment, the tube has a conical wall as one can see in the plan view (figure 13).

In figure 16, the tube-shaped body (31) is cylindrical as better seen in figure 15 and it is sealed by a reversed cup type safety plug (35), threaded inside in order to efficiently anchor to the outside thread (36) of the mouth of the tube. Reference (37) designates the housing for a pressurization and/or leakproof joint (38).

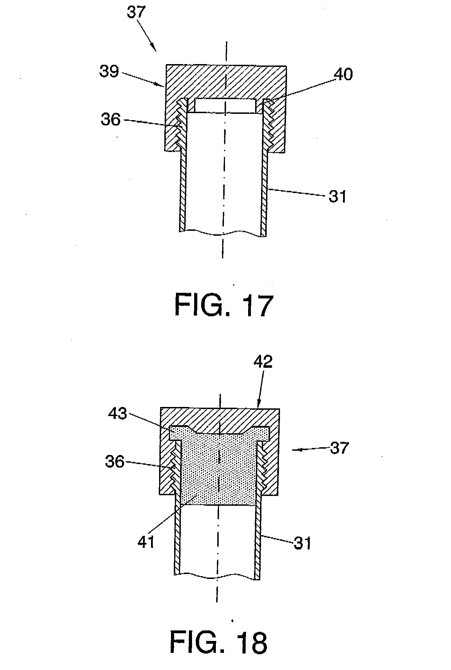

Making special reference to figure 17, the sealing plug is referred to as number (39) and it is similar to the plug (35) of figure 16, but it includes a ring-shaped neck (40) that is inserted in the mouth of the tube.

In figure 18, the cylindrical body threaded outside previously receives the perforable primary plug (41) and afterwards the threaded plug (42) that connects with the thread (36) of the outside of the tube in order to guarantee pressurization. The primary plug (41) has the insertion stop outside flange (43) secured in turn to a recess of the bottom of the plug (42) that also acts as a sealing joint.

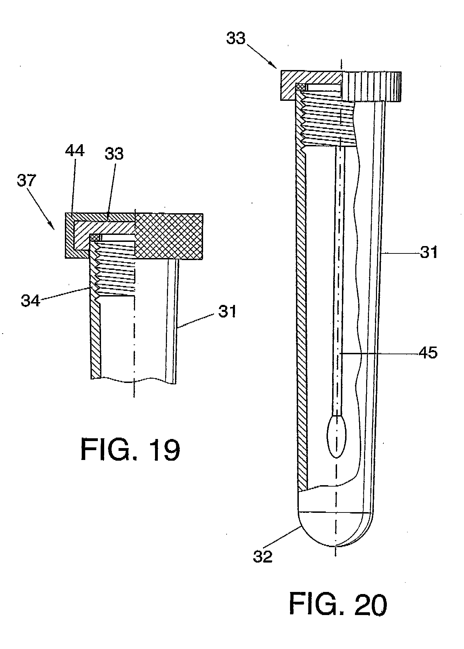

In figure 19, we can see the embodiment wherein the test tube is sealed by the plug (33) and covered with a protective cover (44).

Figure 20 shows a test tube similar to any one of the previous ones and made of course in accordance with the invention, wherein a brush referred to as number (45) has been included and that can be included in any of the plugs of the different embodiments of test tubes that are proposed.

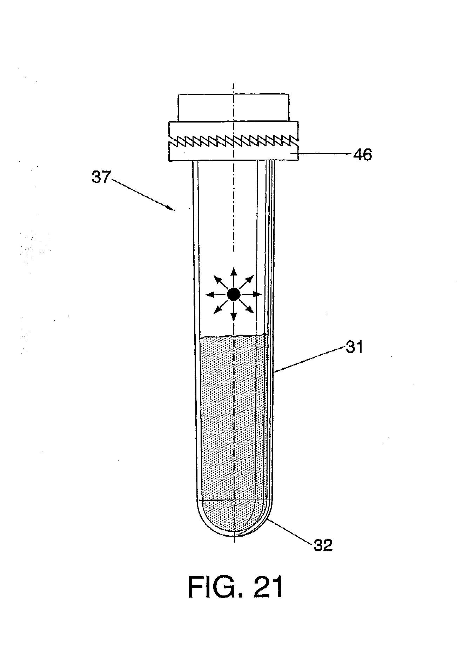

Making special reference to figure 21, a sealing ring designated number (46) connects to the rest of the plug by means of a multitude of radial ribs for tearing and anchoring axially to the outside flange of the end of the tube, or a ring-shaped rib that materializes the anchoring means for the seal.

The pressure "P" that predominates inside the tube and as we have said above can exceed 0.95 bars has been schematically pointed out inside the tube.

## Claims

1. Protective equipment for transporting clinical samples and the like, used to be transported for diagnosis, research or study, included in a rack or similar device, with housings wherein the primary containers or test tubes containing the clinical samples are inserted, **characterized in that** it includes:
- a support or rack (3) where the test tubes (7) containing the samples are inserted;
- a secondary container (2, 2') where the rack (3) with the test tubes (7) will be housed, a secondary container that has any type of sealing device, protecting the inside and the rack (3) holding the test tubes (7);
- a tertiary or outside container (1, 1') with a closing cover (5,5').

2. Protective equipment for transporting clinical samples and the like, according to claim 1, **characterized in that** the tertiary container includes means for keeping the temperature controlled.

3. Protective equipment for transporting clinical samples and the like, according to any one of the preceding claims, **characterized in that** the secondary container is leakproof, with a capacity to endure inside pressure, with a polygonal section, whereas the tertiary or outside container also has a polygonal section with an inside of material in order to dampen impacts for perfect safety of the secondary container, also including cooling devices and document holders, both secondary and tertiary containers are made out of materials capable of enduring autoclaving and/or chemical disinfection.

4. Protective equipment for transporting clinical samples and the like, according to any one of the preceding claims, **characterized in that** the secondary and tertiary containers will have their respective covers in some collapsible parts.

5. Protective equipment for transporting clinical samples and the like, according to any one of claims 1 to 3,
**characterized in that** the secondary and tertiary containers have their respective covers in some collapsible parts.

6. Protective equipment for transporting clinical samples and the like, according to claim 3, **characterized in that** the tertiary container (1) includes a safety valve (17) for differences of pressure when it is used for transport by air or with carbon dioxide snow type refrigerants.

7. Protective equipment for transporting clinical samples and the like, according to any one of claims 3 to 6, **characterized in that** the tertiary container (1) is leakproof when the refrigerant is in the container in bulk form.

8. Protective equipment for transporting clinical samples and the like, according to any one of claims 3 to 7, **characterized in that** the tertiary container (1) includes a traction type seal.

9. Protective equipment for transporting clinical samples and the like, according to any of the claims 3 to 7, **characterized in that** it includes an impact type seal.

10. Protective equipment for transporting clinical samples and the like, according to any of the claims 3 to 7, **characterized in that** it includes a pressure type seal.

11. Protective equipment for transporting clinical samples and the like, according to any one of the claims 3 to 10, **characterized in that** it includes an absorbent material in the secondary container, interposed in the base and/or sides thereof or between the primary container and the secondary container by physical means.

12. Protective equipment for transporting clinical samples and the like, according to any one of the claims 3 to 11, **characterized in that** it includes at least one support for holding documents.

13. Protective equipment for transporting clinical samples and the like, according to claim 1, **characterized in that** the rack comprises a body which has vertical perforations (4) and concave-shape recesses (4'), on one of the side surfaces thereof, covered by a cover type auxiliary part (28) that has an outside flat surface at the same time that said auxiliary part (28) has some recesses or cavities (29) on the larger surface thereof opposite the outside surface, connectable to the surface of the recesses (4'), the body of the rack being able to have a covering area (20) on its perimeter.

14. Protective equipment for transporting clinical samples and the like, according to claim 13, **characterized in that** the rack comprises a monobloc body.

15. Protective equipment for transporting clinical samples and the like, according to claim 13, **characterized in that** the rack is formed by several adjacent parts comprising a general body.

16. Protective equipment for transporting clinical samples and the like, according to any one of claims 13 to 15, **characterized in that** the rack has a prismatic shape without edges.

17. Protective equipment for transporting clinical samples and the like, according to any of claims 13 to 15, **characterized in that** the rack comprises a cylindrical body.

18. Protective equipment for transporting clinical samples and the like, according to any one of claims 13 to 15, **characterized in that** the rack comprises an oval plan view.

19. Protective equipment for transporting clinical samples and the like, according to any one of the claims 13 to 18, **characterized in that** the body of the rack is made out of polypropylene microfibers.

20. Protective equipment for transporting clinical samples and the like, according to any one of claims 13 to 18, **characterized in that** the rack may be made out of fiberglass.

21. Protective equipment for transporting clinical samples and the like, according to any one of claims 13 to 18, **characterized in that** the body of the rack can be made out of polypropylene polyester.

22. Protective equipment for transporting clinical samples and the like, according to any of claims 13 to 18, **characterized in that** the body of the rack may be made out of treated cotton.

23. Protective equipment for transporting clinical samples and the like, according to any one of claims 13 to 18, **characterized in that** the body of the rack may be made out of cellulose, absorbent paper and even non-rigid absorbent materials.

24. Protective equipment for transporting clinical samples and the like, according to claim 1, **characterized in that** each test tube (7) is comprised of a tube-shaped body (31) made of plastic or the like, sealed at one of its ends in a semi-spherical manner (32), and provided with a threaded plug (33, 35, 39, 42), capable of enduring a differential inside pressure of at least 0.95 bars and very strong impacts, capable of including a pressurization and/or leakproof joint (38).

25. Protective equipment for transporting clinical samples and the like, according to claim 24, **characterized in that** the plug (33) has a solid axial extension with an outside thread for connection with the inside thread (34) of the mouth of the tube-shaped body (31).

26. Protective equipment for transporting clinical samples and the like, according to claim 24, **characterized in that** the plug (35) has a cylindrical wall with an inside thread for connection with the outside thread (36) of the mouth of the tube-shaped body (31), the plug (35) including after the thread, a housing for placement of a leakproof seal (38).

27. Protective equipment for transporting clinical samples and the like, according to claim 26, **characterized in that** the bottom of the plug (39) has a coaxial cylindrical neck (40) that fits on the inside mouth of the tube-shaped body (31).

28. Protective equipment for transporting clinical samples and the like, according to claim 24, **characterized in that** the mouth of the tube-shaped body (31) receives inside a primary perforable plug (41), with an insertion stop outside flange (43) that is anchored in an inside recess of the threaded cylindrical wall of a surrounding plug (42) connected to the outside thread (36) of the outside mouth of said tube-shaped body (31).

29. Protective equipment for transporting clinical samples and the like, according to claim 24, **characterized in that** the outside of the plug (33, 35, 39, 42) is covered by a protective cover (44).

30. Protective equipment for transporting clinical samples and the like, according to any one of claims 24 to 29, **characterized in that** the plug (33, 35, 39, 42) is provided with a brush (45) that runs axially through the inside of the tube-shaped body (31).

31. Protective equipment for transporting clinical samples and the like, according to any one of the claims 24 to 30, **characterized in that** the wall of the tube-shaped body (31) is slightly conical, widened toward the mouth.

32. Protective equipment for transporting clinical samples and the like, according to claim 24, **characterized in that** the plug (33, 35, 39 and 42) includes a sealing ring (46) connected to its outside periphery by a multitude of tearing ribs and it is axially anchored to a flange or the like of the outside end of the tube-shaped body (31).

33. Protective equipment for transporting clinical samples and the like, according to claim 2, **characterized in that** the plug (33) has a surrounding cylindrical wall that fits in the outside mouth of the tube-shaped body (31).

34. Protective equipment for transporting clinical samples and the like, according to any of claims 24 to 30 **characterized in that** the wall of the tube-shaped body (31) has a prismatic or polyhedral shape.
